# EUROPEAN PATENT SPECIFICATION

(11) **EP 2 491 853 B1**
(45) Date of publication and mention of the grant of the patent: **09.10.2013**
(21) Application number: 11425045.9
(22) Date of filing: 23.02.2011
(51) Int. Cl.: A61B 1/267, A61M 16/04

(54) **Device comprising a set of magnetic elements, particularly suitable as a support to the procedure of patient's tracheal intubation**
Vorrichtung aus einem Satz magnetischer Elemente, besonders geeignet als Stütze beim Verfahren der Trachealintubation eines Patienten
Dispositif comprenant un ensemble d'éléments magnétiques, convenant particulièrement au support de la procédure d'une intubation trachéale de patients

(43) Date of publication of application: 29.08.2012
(73) Proprietor: De Domenico, Andrea, 00176 Roma (IT)
(72) Inventor: De Domenico, Andrea, 00176 Roma (IT)
(74) Representative: Zizzari, Massimo

(56) References cited:
- WO-A1-2010/079521
- US-A- 4 672 960
- US-B1- 6 878 106

## Description

The present invention concerns a medical device, that is particularly suitable as a support to the procedure of patient's tracheal intubation, and preferably embedded in a blade of a laryngoscope, or an *Airtraq*Ⓡ *video-laryngoscope,* or a *Glidescope*Ⓡ *video-laryngoscope,* or a *LMA*Ⓡ *video-laryngoscope,* or other similar instruments, and that comprises magnetic means able to achieve a guiding and routing system of an endotracheal tube, towards its final position with a far end in trachea.

The *direct laryngoscopy* is a technique that permits to visualize the laryngeal structures in a clear and straight way, and it is achieved by an instrument called *laryngoscope.* This technique made it possible, to anesthetists operating around the world, to define a standard procedure of tracheal intubation in order to place a tube in trachea, by which a patient can receive oxygen and at the same time can have the air ways protected during the anesthesia.

Besides the improvement of such technique, obtained at a global level by years and years of clinical practice, and besides the different shapes of laryngoscopic blades that have been designed and are available on the market, in same cases the direct laryngoscopy is not possible, because of particular anatomical parts in some persons, or because of particular situations of emergency that can occur.

In example, it is not possible when the alignment of the three patient's axis (oral axis, pharyngeal axis and laryngeal axis) cannot be achieved by the standard actions that a physician knows and can put in practice.

Since a very long time, there has been a widely recognized need in order to find some new alternative instruments and techniques, so that an optimal visualization of glottis can be achieved, especially in cases that a direct laryngoscopy results to be not effective or even impossible.

When new technologies became available, with new advanced materials, the laryngeal structures had more chances to be properly visualized, even in a not direct way, by the so called *indirect laryngoscopy.*

Therefore, the techniques of indirect laryngoscopy made it possible to visualize the laryngeal structures clearly, using a camera or an optical fiber, or either a system of prisms with the distal elements placed close to the glottis.

Nowadays, a large set of instruments are available, permitting to handle also difficult situations, and many of these instruments embed some systems based on indirect laryngoscopy.

The important event that caused a big acceleration in progress of this technique, in the field of anesthesiology when diffusion was still at the beginning, surely has been the construction and marketing of optical fibers. When the optical fibers started to be available in the clinical practice, the flexible fiberscope has become the reference instrument applied to all the difficult intubation cases, even considering that some new professional operators had to be trained, said operators coming from the field of experts in anesthesiology, the otolaryngologists and the surgeons specialized in chest. The attempts to design less complex devices, suitable to be used by common anesthetists in the clinical practice, and suitable to provide at the same time the same advantages of optical fibers in indirect laryngoscopy, have led to design of some new special instruments. These are based on standard laryngoscopes, that have a blade which is a part that can move the tongue and to lift/carry the epiglottis, having a particularly curved profile, where an optical fiber is embedded, said fiber reaching the far end of the same blade.

The *Bullard laryngoscope* has been the first of this kind of laryngoscopes, providing the possibility of inspection of laryngeal structures, once the obstacle of the anatomical curve, preventing the direct vision of glottis, has been easily and safely overcome, with a simultaneous precise support of soft tissues, so that an orotracheal intubation can be suddenly achieved.

With reference to devices that, at the present time, permit to achieve the indirect laryngoscopy technique, except the flexible and stiff fiberscope, or *Bonfils fiberscope,* two possible classes can be recognized:
- the *videolaryngoscopes* (Glidescope, McGrath, and C-MAC);
- the *tunneled-systems* (Upscher Scope, *Bullard laryngoscope,* Airtraq, Pentax AWS, and A.P. Advance LMA).

Besides the different visual, optical or video systems, each device belongs to the first or the second class, according to the procedure of using the same device, when the operator can see the laryngeal structures and should insert the endotracheal tube until its proper position in trachea.

Devices in the first class, Glidescope, McGrath, and C-MAC, once the indirect laryngoscopy is possible, according to the blade's shape, usually require that the operator, before to insert the endotracheal tube, has previously and properly fixed its shape. Therefore, the tube's profile is adapted, according to a specific angle, by inserting a stiff stylet inside, so that its final shape allows to address the tube beyond the tongue, and towards the patient's trachea.

Instead, devices in the second class, like i.e. Airtraq, Pentax AWS, and A.P. Advance LMA, contain the endotracheal tube in a tunnel that is integrated in the blade, so that a guidance can be achieved all along the path, until the tube exits directly with the far end at the proper position in trachea. Therefore, the procedure does not require the operator to use a stylet, and does not require to maneuver the endotracheal tube either, it just requires the tube to be pushed inside a stiff driving channel.

Although this solution prevents possible risks arising from the use of stylets, and therefore represents a more comfortable procedure of orotracheal intubation, according to an indirect laryngoscopy technique, however it is characterized by significant drawbacks.

First of all, the operator is forced to express some strong actions of pulling and rotating the device, and that's just an attempt, sometimes not effective, to align properly the far end of the instrument to the tracheal axis.

Furthermore, the requirement to insert the endotracheal tube in an operative channel, in order to follow a proper driving path, leads as a consequence to the definition of an increased instrument's thickness, and to an increased minimum inter-foreteeth distance, so that the same instrument can enter into the patient's mouth, and/or leads to the use of different instruments of different size, according to the diameter of the tube to be inserted.

Document WO 2010/079521 discloses a support device comprising a set of magnetic elements placed along the longitudinal axis of a laryngoscope blade. These magnetic elements interact with a metallic spiral tube attached to an endotracheal tube.

Therefore, the subject of the present invention consists of a device overcoming all the previous drawbacks, and achieving a great versatility of use, because it can be embedded into large part of the available systems supporting the orotracheal intubation actually on the market, like i.e.: laryngoscopes, video-laryngoscopes, tunneled video-laryngoscopes, etc.

In particular, the device of this invention achieves a guiding and routing system for an endotracheal tube by use of magnetic means, without requiring a direct operative channel especially designed for that, and without requiring a use of a stylet, preventing therefore possible risks of damages for the patient, arising from the same instrument.

Furthermore, the far end of this device includes an articulated joint mechanism, that is useful in case of need to move tissues and anatomical inner parts of patient, or just in case it is necessary to change the curvature of the blade at the distal part, in order to adapt the instrument's shape to the clinical circumstances; or further, in case it is necessary to change the curvature of the blade and at the same time it is necessary that the endotracheal tube follows this new curvature, at the moment when it is pushed down along the profile of the blade, so that the best alignment of distal end of the endotracheal tube and tracheal axis can be achieved.

The most significant characteristic of the present invention is to include a fine tuning of the position of magnetic means, so that it can be fixed the "escape angle" of the endotracheal tube from the guiding and routing system, and the intubation procedure can be adapted to different operative circumstances, especially in cases where a direct or indirect visual perception of the situation is possible. Another significant characteristic of the invention is that the same magnetic elements (double function of the magnetic platform) can be used not only as a guiding and routing system for the endotracheal tube, but as a set of elements mechanically connected to the distal and articulated segment of blade, so that is possible to increase the curvature of both blade and endotracheal tube, if necessary.

The disclosed device permits therefore to achieve a significant decrease of thickness, that define the minimal inter-foreteeth distance in order to use the actual tunneled laryngoscopes, so that they can be inserted in a patient's mouth, a variable routing direction at each step of progress, under control by a tuning mechanism, a more comfortable procedure that does not require any use of a stylet to define shape and profile, and the possibility to move the distal end by an articulated joint mechanism, that is under control using the same guiding and routing system for the endotracheal tube.

Therefore, it is specific subject of the present invention a device particularly suitable as a support to the procedure of a patient's tracheal intubation, and preferably embedded in a blade of a laryngoscope, or an *Airtraq*Ⓡ *video-laryngoscope,* or a *Glidescope*Ⓡ *video-laryngoscope,* or a *LMA*Ⓡ *video-laryngoscope,* or other similar instruments, the device comprising:
- a first set of magnetic elements that are all connected each other, by a first flexible/articulated supporting structure, in order to achieve a so called *magnetic platform,* free to move forward or backward, under control of an operator, along the longitudinal direction inside an operative channel belonging to the same device;
- a second set of magnetic elements that are all connected each other, by a second flexible supporting structure, in order to achieve a so called *magnetic train,* previously inserted in an endotracheal tube; elements of *magnetic train* have an opposite polarity in respect of elements of the *magnetic platform,* so that a magnetic attractive force is generated by their interaction for all the time, keeping said endotracheal tube strictly in contact with said *magnetic platform,* so that the same endotracheal tube can move, forward or backward, under control of an operator, along the longitudinal profile of said operative channel, in order to achieve a guiding and routing system for the endotracheal tube, because the tube follows a curved trajectory and it extends itself with a far end aligned to a direction that follows the tangent to the *magnetic platform* profile, at the last point of magnetic contact.

The present invention will now be described for illustrative but not limitative purposes, according to its preferred embodiments, with particular reference to figures of the enclosed drawings, wherein:
figure 1 is a front perspective view of a device with magnetic elements, according to the present invention, preferably embedded in a *Airtraq*Ⓡ *video-laryngoscope;*
figure 2 is a lateral view of a device with magnetic elements embedded in a *Airtraq*Ⓡ *video-laryngoscope,* like that of figure 1, inserted in the upper part of a patient, related to mouth and first tract of air ways, the same patient being represented according to a lateral sectioned view;
figures 3, 4 and 5 are a sequence of front perspective views of the same device of figure 1, and of an endotracheal tube that is moved in contact with the so called *magnetic platform* part, and that is represented at three different positions;
figures 6, 7 and 8 are a sequence of lateral perspective views of the same device of figure 1, where the so called *magnetic platform* is moved forward, and it is represented at three different positions;
figure 9 is a front perspective view of a device with magnetic elements, according to the present invention, preferably embedded in a *standard* laryngoscope;
figure 10 is a lateral view of a device with magnetic elements embedded in a standard laryngoscope, like that of figure 9, inserted in the upper part of a patient, related to mouth and first tract of air ways, the same patient being represented according to a lateral sectioned view;
figure 11 is a front perspective view of a device with magnetic elements, according to the present invention, preferably embedded in a *LMA*Ⓡ *video-laryngoscope;*
figure 12 is a front perspective view of a device with magnetic elements, according to the present invention, preferably embedded in a *LMA*Ⓡ *video-laryngoscope,* where position of *magnetic platform* can be moved under control of an operator;
figure 13 is a lateral view of a device with magnetic elements embedded in a *LMA*Ⓡ *video-laryngoscope,* like that of figure 11, inserted in the upper part of a patient, related to mouth and first tract of air ways, the same patient being represented according to a lateral sectioned view;
figure 14 is a lateral view of a device with magnetic elements embedded in a *Airtraq*Ⓡ *video-laryngoscope,* like that of figure 1, where the forward position of *magnetic platform* defines an outgoing angle α of the endotracheal tube;
figure 15 is a lateral view of a device with magnetic elements embedded in a *Airtraq*Ⓡ *video-laryngoscope,* like that of figure 1, where the backward position of magnetic platform defines an outgoing angle β of the endotracheal tube;
figure 16 is a front view of a device with magnetic elements embedded in a *Airtraq*Ⓡ *video-laryngoscope,* like that of figures 14 and 15, where it is shown the presence of an optical visual channel;
figure 17 is a front view of a detail of figure 16, with reference to the distal end of the outgoing part of an endotracheal tube, with respective optical lighting and inspection means of a *Airtraq*Ⓡ *video-laryngoscope.*

It is here underlined that only few of the many conceivable embodiments of the present invention are described, which are just some specific non-limiting examples, having the possibility to describe many other embodiments based on the disclosed technical solutions of the present invention.

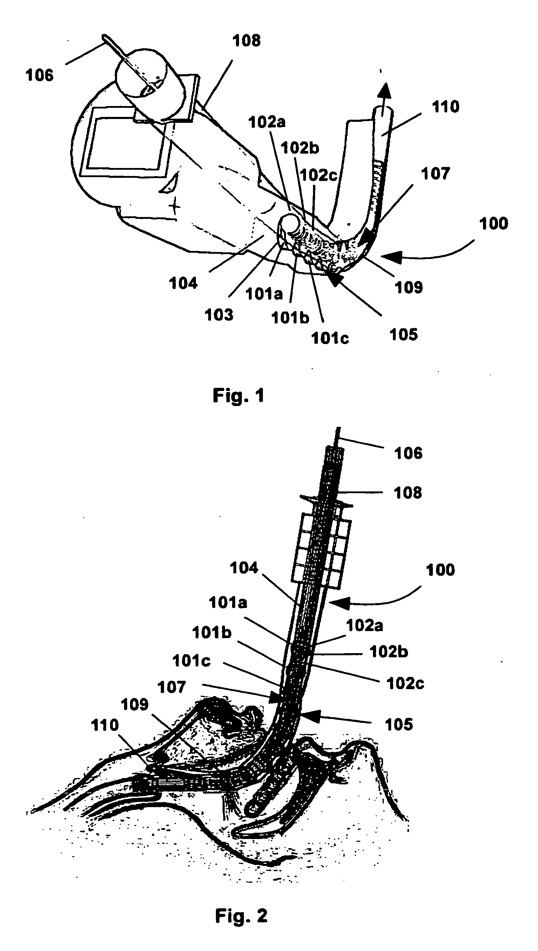

In figure 1 it is illustrated a device with magnetic elements 100, particularly suitable as a support to the procedure of tracheal intubation of a patient, embedded in the blade of a *Airtraq*Ⓡ *video-laryngoscope.* The blade of the same laryngoscope, in the prior art has a longitudinal profile with some protruding walls, that achieves a guiding and routing tunnel, along which an endotracheal tube 108 is inserted until its final position, with the distal end in trachea, as illustrated in figure 2.

Instead, device 100 of the present invention is characterized by having the protruding walls removed, and the guiding function is achieved through a *magnetic platform* 105, that is placed into the blade, and that interacts with a *magnetic train* 107, that has been previously and steadily inserted within an endotracheal tube 108.

The *magnetic platform* 105 comprises a first set of magnetic elements 101 a, 101 b, ..., etc., that are all connected each other, by a first flexible/articulated supporting structure 103; the *magnetic train* 107 comprises instead a second set of magnetic elements 102a, 102b, ..., etc., that are all connected each other, by a second flexible supporting structure 106. The elements 102a, 102b, ..., etc., of the *magnetic train* 107 have an opposite polarity in respect of elements 101 a, 101 b, ... , etc., of said *magnetic platform* 105, so that a magnetic attractive force is generated by interaction all the time, keeping said endotracheal tube 108 strictly in contact with said *magnetic platform* 105.

The *magnetic platform* 105 is free to move forward or backward, under control of an operator, along the longitudinal direction of an operative channel 104 belonging to the same device 100. Furthermore, the *magnetic platform* 105 can be completely removed from its position inside the operative channel 104, when said structure 103 (after disconnection of gear wheel 120) is strongly pulled out by operator, so that the entire device 100 can be removed in a very safe way and without trauma for patient, once the endotracheal tube 108 has been properly inserted in trachea.

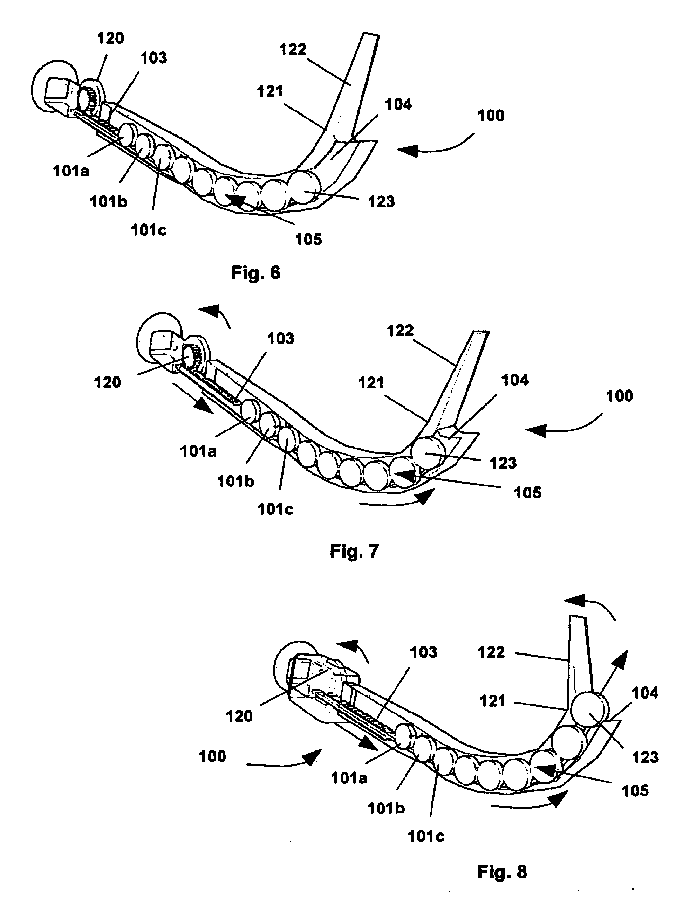

Figures 6, 7 and 8 show a sequence where the so called *magnetic platform* 105 is moved, and it is represented at three different positions.

The supporting structure 103 of the *magnetic platform* 105 is composed of a set of elements, that connect all respective magnetic elements 101a, 101b, ... , etc., and said set of elements are stiff in the longitudinal direction and articulated laterally. The same structure 103 ends, at the closest part to the human operator, with a gear wheel 120; when the operator makes the wheel 120 to rotate backward, the *magnetic platform* 105 goes ahead deep in the blade, when the operator makes the wheel 120 to rotate forward, the *magnetic platform* 105 goes back to the closest part to operator. The gear wheel 120 includes a special stopping/blocking system that makes it impossible, if activated, the *magnetic platform* 105 to move along the operative channel 104.

The supporting structure 106 of the *magnetic train* 107 is composed of a set of elements, that connect all respective magnetic elements 102a, 102b, ..., etc., and said set of elements are stiff in the longitudinal direction and articulated laterally. When the operator pushes the structure 106 forward, the *magnetic train* 107 goes ahead into the endotracheal tube 108, when the operator pushes the structure 106 backward, the *magnetic train* 107 goes back from the endotracheal tube 108, to the closest part to operator.

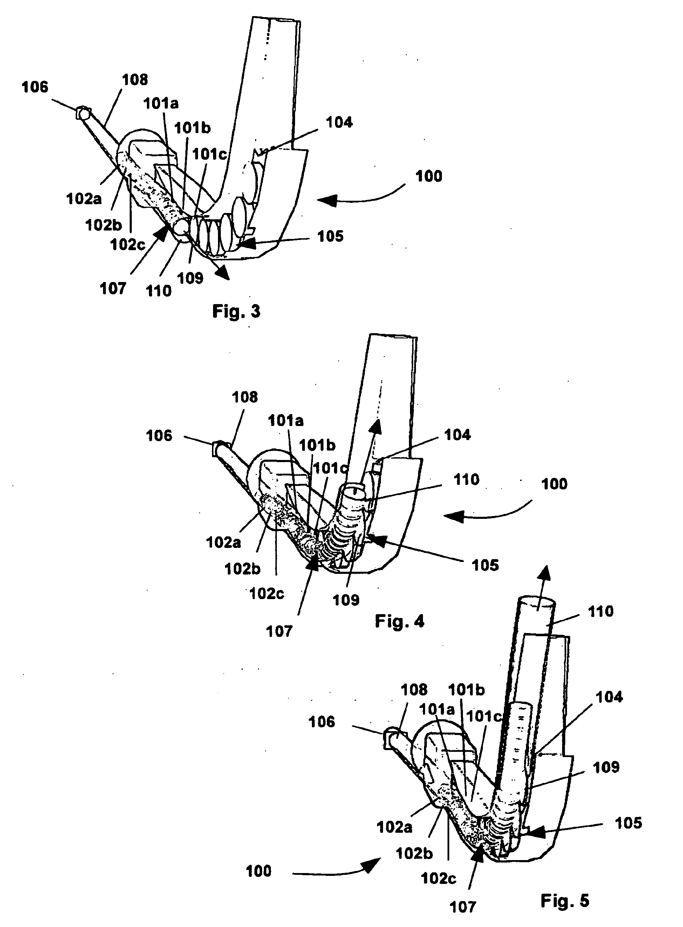

Figures 3, 4 and 5 show a sequence where an endotracheal tube 108 is moved in contact with the *magnetic platform* 105, and it is represented at three different positions.

An operator can move the endotracheal tube 108, forward or backward, along the longitudinal profile of said operative channel 104, in order to achieve a guiding and routing system of the same endotracheal tube 108, because the tube follows a respective curved trajectory and it extends itself with its far end 110 aligned to a direction that follows the tangent to said operative channel's 104 profile, at the last point of magnetic contact 109.

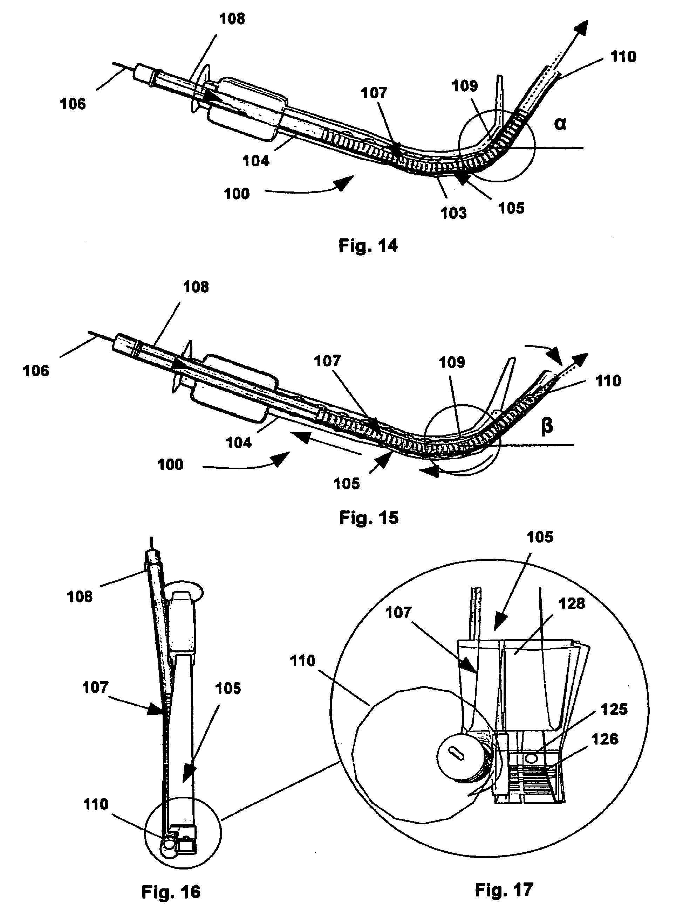

Figure 14 shows that a forward position of *magnetic platform* 105, with a forward last point of magnetic contact 109, defines an outgoing angle α of the endotracheal tube that is very diverging in respect to an horizontal line; instead, figure 15 shows that a backward position of *magnetic platform* 105, with a backward last point of magnetic contact 109, defines an outgoing angle β < α of the endotracheal tube 108 that is less diverging in respect to the same horizontal line.

The elements 101a, 101b, ..., etc., of the *magnetic platform* 105 have a cylindrical shape and are placed transversally in respect to the longitudinal direction of said operative channel 104 where they are inserted, so that they can roll inside, and so that they achieve a little friction of *magnetic platform* 105 during its motion.

The elements 102a, 102b, ..., etc., of the *magnetic train* 107 have a cylindrical shape and are placed coaxially in respect to the longitudinal direction of said endotracheal tube 108 where they are inserted, so that they can change direction in order to follow a curved shape of the endotracheal tube 108, when it follows a specific path in contact with the *magnetic platform* 105.

The first set of magnetic elements 101a, 101b, ..., etc., can be entirely composed of magnetic elements, otherwise can be composed of an alternation of magnetic elements and magnetically neutral elements, i.e. made of plastic, so that the latter ones have as unique function to keep at a specific distance the elements of said *magnetic platform* 105.

The second set of magnetic elements 102a, 102b, ..., etc., can be entirely composed of magnetic elements, otherwise can be composed of an alternation of magnetic elements and magnetically neutral elements, i.e. made of plastic, so that the latter ones have as unique function to keep at a specific distance the elements of said *magnetic train* 107.

The elements 101a, 101b, ..., etc., of the *magnetic platform* 105, and the elements 102a, 102b, ..., etc., of the *magnetic train* 107, can be composed of permanent magnets or electromagnets with a related power supply circuit, and batteries with on/off switches and tuning devices.

According to another embodiment of the invention, the far end 122 of the blade of device 100 is articulated, and can rotate clockwise or counterclockwise in respect to a transversal pivot 121. The last element 123 of the *magnetic platform* 105 is composed of a magnetically neutral element, i.e. made of plastic, so that a motion forward of the *magnetic platform* 105 makes the last element 123 to push against the element 122, and so that the same element 122 is lifted up and allows the displacement of patient's tissues and/or other anatomical parts - this is called *McCoy function.* The same element 122 comprises a return spring, that permits the element 122 to go back at its initial position, when the *magnetic platform* 105 is moved backward and the element 123 stops to make a pressure on the side, and goes back at its initial position either. An elastic membrane of protection, placed at the terminal part of the operative channel 104, achieves two results: first, it permits to at least two elements belonging to the *magnetic platform* 105 to go outside from the distal part of the operative channel 104; and second, it keeps the *magnetic platform* 105 sterilized, so that it can be used on another blade that is disposable, or on similar means using this type of technology.

The device 100 of the present invention can comprise, as accessories, a first disposable covering, preferably made of plastics and sterilized, that covers the blade of device 100 and all the *magnetic platform* 105, and a second disposable covering, either preferably made of plastics and sterilized, that covers all the *magnetic train 107,* placed inside the endotracheal tube 108, so that to prevent possible infections and/or contaminations. Indeed, the device 100 itself can be disposable, with reference to the entire blade and channels 104 and 128, including the *magnetic platform* 105 and further video or optical devices eventually embedded.

The device 100 of the invention can be easily integrated with the optical visual means, typical of a *Airtraq*Ⓡ *video-laryngoscope.*

In example, figures 16 and 17 show said operative channel 104, where said *magnetic platform* 105 can move inside, is exactly parallel to an optical visual channel 128, through which the visual signals of the detected image are transmitted, from the far end of device 100 to the view of said human operator. Furthermore, it is possible that visual channel 128 is just an empty channel, where visual systems like a fiberscope, or other available devices, can be inserted.

Furthermore, some embodiments of the invention disclose that said operative channel 104 is placed at the right side, from the point of view of an operator, in respect to said optical visual channel 128, or at the left side in order to make it comfortable to left-handed operators. Then, another embodiment of invention discloses that said operative channel 104 and said optical visual channel 128 are placed side by side, with the endotracheal tube 108 placed in the space between.

In such a way, the patient's anatomical parts result to be better protected, in respect to situations where the endotracheal tube 108 could touch the tongue, and the tissues can be entrapped between the same endotracheal tube 108 and the elements of device 100, leading as a consequence to risks of abrasions and inner scratching to the patient.

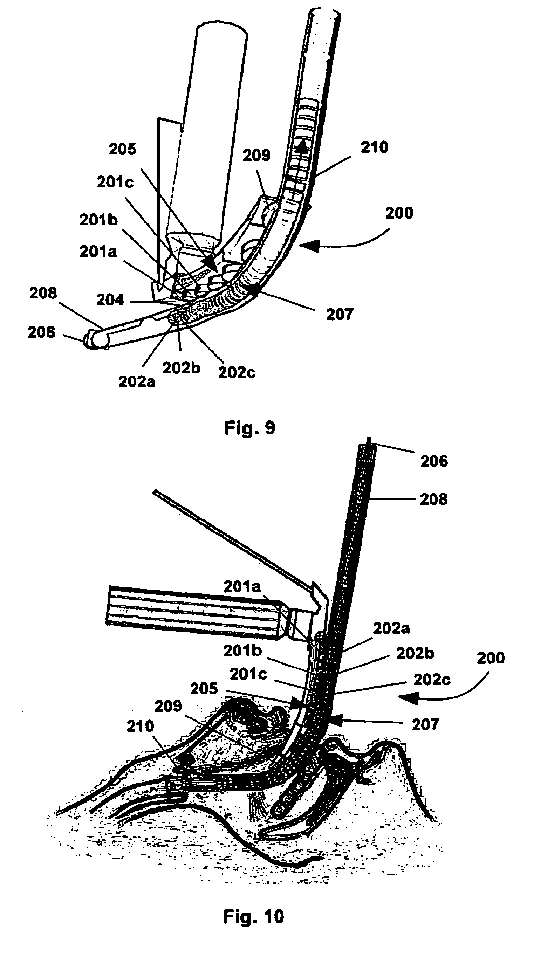

Figure 9 shows a device with magnetic elements 200, preferably embedded in a *standard* laryngoscope. The guiding function is achieved through a *magnetic platform* 205, that is placed at the bottom of the blade, and that interacts with a *magnetic train* 207, that has been previously and steadily inserted within an endotracheal tube 208.

The *magnetic platform* 205 comprises a first set of magnetic elements 201 a, 201 b, ... , etc., that are all connected each other, directly to the blade of laryngoscope; the *magnetic train* 207 comprises instead a second set of magnetic elements 202a, 202b, ..., etc., that are all connected each other, by a flexible supporting structure 206. The elements 202a, 202b, ..., etc., of the *magnetic train* 207 have an opposite polarity in respect of elements 201a, 201 b, ... , etc., of said *magnetic platform* 205, so that a magnetic attractive force is generated by interaction all the time, keeping said endotracheal tube 208 strictly in contact with said *magnetic platform* 205.

An operator can move the endotracheal tube 208, forward or backward, along the longitudinal profile of the blade of laryngoscope, in order to achieve a guiding and routing system of the same endotracheal tube 208, because the tube follows a respective curved trajectory and it extends itself with its far end 210 aligned to a direction that follows the tangent to the blade's profile, at the last point of magnetic contact 209.

Therefore, the device with magnetic elements 200 achieves a guiding and driving path, along which an endotracheal tube 208 is pushed up to its final position, with the distal end in trachea, as illustrated in figure 10.

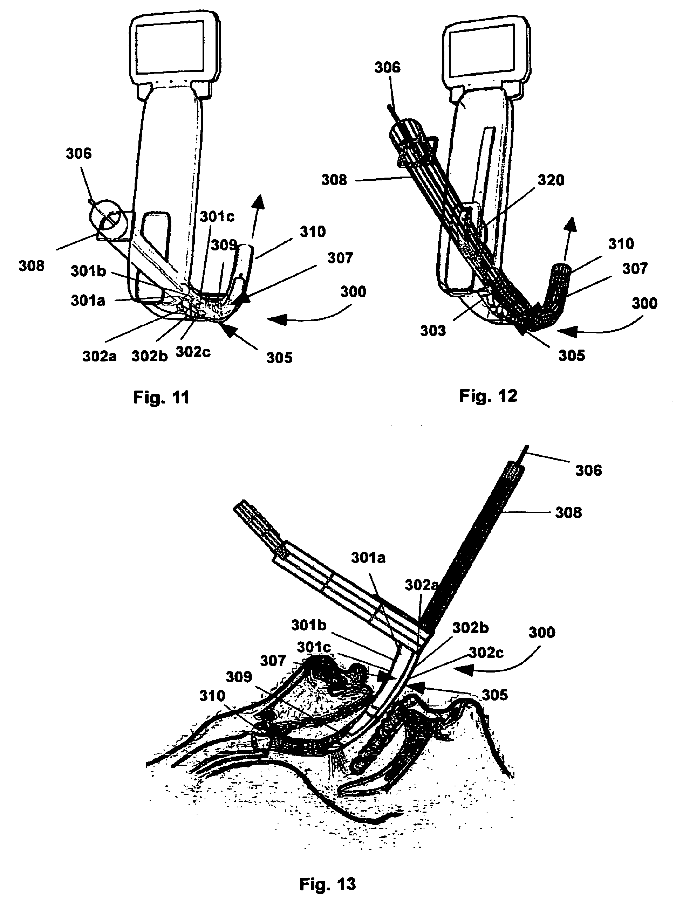

Figure 11 shows a device with magnetic elements 300, preferably embedded in *a LMA*Ⓡ *video-laryngoscope.* The guiding function is achieved through a *magnetic platform* 305, that is placed at a lateral side of the blade, and that interacts with a *magnetic train* 307, that has been previously and steadily inserted within an endotracheal tube 308.

The *magnetic platform* 305 comprises a first set of magnetic elements 301 a, 301 b, ..., etc., that are all connected each other, and that are placed: inside the same structure housing the video or optical devices of laryngoscope and supporting the disposable and interchangeable blade; or inside the same disposable blades that are assembled on said structure according to different clinical circumstances; the *magnetic train* 307 comprises instead a second set of magnetic elements 302a, 302b, ..., etc., that are all connected each other, by a flexible supporting structure 306. The elements 302a, 302b, ..., etc., of the *magnetic train* 307 have an opposite polarity in respect of elements 301a, 301b, ..., etc., of said *magnetic platform* 305, so that a magnetic attractive force is generated by interaction all the time, keeping said endotracheal tube 308 strictly in contact with said *magnetic platform* 305.

As shown in figure 12, the *magnetic platform* 305 is free to move forward or backward, under control of an operator, along the longitudinal direction of an operative channel 304 belonging to the same device 300.

The supporting structure 303 of the *magnetic platform* 305 is composed of a set of elements, that connect all respective magnetic elements 301 a, 301 b, ... , etc., and said set of elements are stiff in the longitudinal direction and articulated laterally. The same structure 303 ends, at the closest part to the human operator, with a gear wheel 320; when the operator makes the wheel 120 to rotate backward, the *magnetic platform* 305 goes ahead deep in the blade, when the operator makes the wheel 320 to rotate forward, the *magnetic platform* 305 goes back to the closest part to operator.

The operator can move the endotracheal tube 308, forward or backward, along the longitudinal profile of the blade of laryngoscope, in order to achieve a guiding and routing system of the same endotracheal tube 308, because the tube follows a respective curved trajectory and it extends itself with its far end 310 aligned to a direction that follows the tangent to the blade's profile, at the last point of magnetic contact 309.

Therefore, the device with magnetic elements 300 achieves a guiding and routing path, along which an endotracheal tube 308 is pushed up to its final position, with the distal end in trachea, as illustrated in figure 13.

Either in this case, the device 300 of the present invention can comprise, as accessories, a first disposable covering, preferably made of plastics and sterilized, that covers the blade (or the structure supporting the blade) of device 300 and all the *magnetic platform* 305, and a second disposable covering, either preferably made of plastics and sterilized, that covers all the *magnetic train* 307, placed inside the endotracheal tube 308, so that to prevent possible infections and/or contaminations.

Therefore, the above examples show that the present invention achieves all the proposed objectives. In particular, it discloses a device that permits to overcome all the drawbacks of the prior art, achieving a great versatility of use, because it can be embedded into large part of the available systems supporting the orotracheal intubation actually on the market, like i.e.: laryngoscopes, video-laryngoscopes, tunneled video-laryngoscopes, etc.

In particular, the device of this invention achieves a guiding and routing system for an endotracheal tube by use of magnetic means, without requiring a direct operative channel especially designed for that, and without requiring a use of a stylet, preventing therefore possible risks of damages for the patient, arising from the same instrument.

Furthermore, the far end of this device includes an articulated joint mechanism, that is useful in case of need to move tissues and anatomical inner parts of patient, or just in case it is necessary to change the curvature of the blade at the distal part, in order to adapt the instrument's shape to the clinical circumstances; or further, in case it is necessary to change the curvature of the blade and at the same time it is necessary that the endotracheal tube follows this new curvature, at the moment when it is pushed down along the profile of the blade, so that the best alignment of distal end of the endotracheal tube and tracheal axis can be achieved.

The most significant characteristic of the present invention is to include a fine tuning of the position of magnetic means, so that it can be fixed the "escape angle" of the endotracheal tube from the guiding and routing system, and the intubation procedure can be adapted to the different operative circumstances, especially in cases where a direct or indirect visual perception of the situation is possible. Another significant characteristic of the invention is that the same magnetic elements (double function of the magnetic platform) can be used not only as a guiding and routing system for the endotracheal tube, but as a set of elements mechanically connected to the distal and articulated segment of blade, so that is possible to increase the curvature of both blade and endotracheal tube, if necessary.

The disclosed device permits therefore to achieve a significant decrease of thickness, that define the minimal inter-foreteeth distance in order to use the actual tunneled laryngoscopes, so that they can be inserted in a patient's mouth, a variable routing direction at each step of progress, under control by a tuning mechanism, a more comfortable procedure that does not require any use of a stylet to define shape and profile, and the possibility to move the distal end by an articulated joint mechanism, that is under control using the same guiding and routing system for the endotracheal tube.

The present invention has been described for illustrative but not limitative purposes, according to its preferred embodiments, but it is clear that modifications and/or changes can be introduced by those skilled in the art without departing from the relevant scope, as defined in the enclosed claims.

## Claims

1. Device (100), particularly suitable as a support to procedure of patient's tracheal intubation, and preferably embedded in a blade of a laryngoscope or other similar instruments, the device comprising:
- a first set of magnetic elements (101a, 101b, ..., etc.) that are all connected to each other, by a first flexible/articulated supporting structure (103), in order to achieve a so called *magnetic platform* (105), free to move forward or backward, under control of an operator, along the longitudinal direction of an operative channel (104) belonging to the device (100);
- a second set of magnetic elements (102a, 102b, ..., etc.) that are all connected to each other, by a second flexible supporting structure (106), in order to achieve a so called *magnetic train* (107), previously and steadily inserted in an endotracheal tube (108); elements (102a, 102b, ..., etc.) of *magnetic train* (107) having an opposite polarity in respect of elements (101a, 101b, ..., etc.) of said *magnetic platform* (105), so that a magnetic attractive force is generated by interaction all the time, keeping said endotracheal tube (108) strictly in contact with said *magnetic platform* (105),
so that the endotracheal tube (108) can move, forward or backward, under control of an operator, along the longitudinal profile of said operative channel (104), in order to achieve a guiding and routing system of the endotracheal tube (108), because the tube follows a respective curved trajectory and it extends itself with its far end (110) aligned to a direction that follows the tangent to said *magnetic platform* (105) profile, at the last point of magnetic contact (109).

2. Device (100), according to the previous claim, **characterized in that**:
- said first set of magnetic elements (101a, 101b, ..., etc.) is entirely composed of magnetic elements, otherwise is composed of an alternation of magnetic elements and magnetically neutral elements, i.e. made of plastic, so that the latter ones have as unique function to keep at a specific distance the elements of said *magnetic platform* (105).

3. Device (100) according to the previous claim, **characterized in that**:
- said second set of magnetic elements (102a, 102b, ..., etc.) is entirely composed of magnetic elements, otherwise is composed of an alternation of magnetic elements and magnetically neutral elements, i.e. made of plastic, so that the latter ones have as unique function to keep at a specific distance the elements of said *magnetic train* (107).

4. Device (100) according to one or more of the previous claims, **characterized in that**:
- said elements (101a, 101b, ..., etc.) of the *magnetic platform* (105) have a cylindrical shape and are placed transversally in respect to the longitudinal direction of said operative channel (104) where they are inserted, so that they can roll inside, and so that they achieve a little friction of *magnetic platform* (105) during its motion.

5. Device (100) according to one or more of the previous claims, **characterized in that**:
- said elements (102a, 102b, ..., etc.) of the *magnetic train* (107) have a cylindrical shape and are placed coaxially in respect to the longitudinal direction of said endotracheal tube (108) where they are inserted, so that they can change direction in order to follow a curved shape of the endotracheal tube (108), when it follows a specific path in contact with the *magnetic platform* (105).

6. Device (100) according to one or more of the previous claims, **characterized in that**:
- said supporting structure (103) of the *magnetic platform* (105) is composed of a set of elements, that connect all respective magnetic elements (101a, 101b, ..., etc.) and said set of elements are stiff in the longitudinal direction and articulated laterally; the same structure (103) ends, at the closest part to the human operator, with a gear wheel (120); when the operator makes the wheel (120) to rotate backward, the *magnetic platform* (105) goes ahead deep in the blade, when the operator makes the wheel (120) to rotate forward, the *magnetic platform* (105) goes back to the closest part to operator.

7. Device (100) according to one or more of the previous claims, **characterized in that**:
- said supporting structure (106) of the *magnetic train* (107) is composed of a set of elements, that connect all respective magnetic elements (102a, 102b, ..., etc.) and said set of elements are stiff in the longitudinal direction and articulated laterally; when the operator pushes the structure (106) forward, the *magnetic train* (107) goes ahead into the endotracheal tube (108), when the operator pushes the structure (106) backward, the *magnetic train* (107) goes back from the endotracheal tube (108), to the closest part to operator.

8. Device (100) according to one or more of the previous claims, **characterized in that**:
- the far end (122) of the blade of device (100) is articulated, and can rotate clockwise or counterclockwise in respect to a transversal pivot (121); the last element (123) of the *magnetic platform* (105) is composed of a magnetically neutral element, i.e. made of plastic, so that a motion forward of the *magnetic platform* (105) makes the last element (123) to push against the side of element (122), and so that the same element (122) is lifted up and allows the displacement of patient's tissues and/or other anatomical parts; the same element (122) comprises a return spring, that permits the element (122) to go back at its initial position, when the *magnetic platform* (105) is moved backward and the element (123) stops to make a pressure on element (122), and goes back at its initial position either; an elastic membrane of protection, placed at the terminal part of the operative channel (104), achieves two results: first, it permits to at least two elements belonging to the *magnetic platform* (105) to go outside from the distal part of the operative channel (104); and second, it keeps the *magnetic platform* (105) sterilized, so that it can be used on another blade that is disposable, or on similar means using this type of technology.

9. Device (100) according to one or more of the previous claims, **characterized in that**:
- said elements (101a, 101b, ..., etc.) of the *magnetic platform* (105) are composed of permanent magnets or electromagnets with a related power supply circuit, and batteries with on/off switches and tuning devices;
- said elements (102a, 102b, ..., etc.) of the *magnetic train* (107) are composed of permanent magnets or electromagnets with a related power supply circuit, and batteries with on/off switches and tuning devices.

10. Device (100) according to one or more of the previous claims, **characterized in that** further comprising:
- a first disposable covering, preferably made of plastics and sterilized, that covers the blade of device (100) and all the *magnetic platform* (105), so that to prevent possible infections and/or contaminations; the device (100) itself can be disposable,
- a second disposable covering, preferably made of plastics and sterilized, that covers all the *magnetic train* (107), placed inside the endotracheal tube (108), so that to prevent possible infections and/or contaminations.

11. Device (100) according to one or more of the previous claims, **characterized in that**:
- said operative channel (104), where said *magnetic platform* (105) can move inside, is exactly parallel to an optical visual channel (128), through which visual signals of a detected image are transmitted, from the far end of device (100) to the view of said human operator.

12. Device (100) according to claim 11, **characterized in that**:
- said operative channel (104) is placed at the right side, from the point of view of an operator, in respect to said optical visual channel (128), or at the left side in order to make it comfortable to left-handed operators.

13. Device (100) according to to claim 11, **characterized in that**:
- said operative channel (104) and said optical visual channel (128) are placed side by side, with the endotracheal tube (108) placed in the space between,
so that the patient's anatomical parts result to be better protected, in respect to situations where the endotracheal tube (108) could touch the tongue, and the tissues can be entrapped between the same endotracheal tube (108) and the elements of device (100), leading as a consequence to risks of abrasions and inner scratching to the patient.

14. Device (100) according to claim 6, **characterized in that**:
- said *magnetic platform* (105) can be completely removed from its position inside the operative channel (104), when said structure (103) is strongly pulled out by operator, after disconnection of gear wheel (120),
so that the entire device (100) can be removed in a very safe way and without a trauma for patient, once the endotracheal tube (108) has been properly inserted in trachea.

15. Device (100) according to claim 6, **characterized in that**:
- said gear wheel (120) includes a special stopping/blocking system that makes it impossible, if activated, to move the *magnetic platform* (105) along the operative channel (104).

## Patentansprüche

1. Gerät (100), das insbesondere als Support des Verfahrens der trachealen Intubation eines Patienten geeignet ist und vorzugsweise an der Klinge eines Laryngoskops angebracht ist, umfasst folgendes:
- eine erste Reihe von magnetischen Elementen (101a, 101b, ..., usw.), die alle über einer erste flexible/gelenkige Trägerstruktur (103) miteinander verbunden sind, um so eine sogenannte *magnetische Plattform* (105) zu bilden, die sich unter Aufsicht eines Bedieners entlang der Längsrichtung in einem Betriebskanal (104), der zum Gerät (100) gehört, frei vorwärts oder rückwärts, bewegen kann;
- eine zweite Reihe von magnetischen Elementen (102a, 102b, ..., usw.), die alle über einer zweite flexible Trägerstruktur (106) miteinander verbunden sind, um so eine sogenannte *magnetische Bahn* (107) zu bilden, die zuvor ins Innere eines Endotrachealtubus (108) eingefügt wurde; die Elemente (1 02a, 102b, ..., usw.) der *magnetische Bahn* (107) haben eine entgegengesetzten Polarität zu den Elementen (101a, 101b, ..., usw.) der *magnetische Plattform* (105), so dass aus ihrer Wechselwirkung die gesamte Zeit eine magnetische Anziehungskraft erzeugt wird, die den Endotrachealtubus (108) immer in Kontakt mit der *magnetische Plattform* (105) hält,
so, dass sich unter Aufsicht eines Bedieners der Endotrachealtubus (108) entlang dem gesamten Längsprofil des Betriebskanals (104), der daher ein Führungs- und Leitwegsystem für den Endotrachealtubus (108) bildet, vorwärts oder rückwärts bewegen kann, damit letzterer den Biegungen folgt und am Ausgang mit den distalen Enden (110) in der Tangentenrichtung an seinem letzten magnetischen Kontaktpunkt (109) zum Profil des Betriebskanals (104) ausgerichtet ist.

2. Gerät (100), das gemäß dem vorherigen Anspruch **dadurch gekennzeichnet ist, dass**:
- die obengenannte erste Reihe von magnetischen Elementen (101a, 101b, ..., usw.) vollständig aus magnetischen Elementen besteht oder aus einem Wechsel von magnetischen Elementen und Elementen aus einem magnetisch neutralen Material, z.B. Kunststoff besteht, wobei letztere einzig die Funktion haben, mechanisch die Elemente der obengenannten *magnetischen Plattform* (105) zu distanzieren.

3. Gerät (100), das gemäß dem vorherigen Anspruch **dadurch gekennzeichnet ist, dass**:
- die obengenannte zweite Reihe von magnetischen Elementen (102a, 102b, ..., usw.) vollständig aus magnetischen Elementen besteht oder aus einem Wechsel von magnetischen Elementen und Elementen aus einem magnetisch neutralen Material, z.B. Kunststoff besteht, wobei letztere einzig die Funktion haben, mechanisch die Elemente die obengenannte *magnetische Bahn* (107) zu distanzieren.

4. Gerät (100), das gemäß einem oder mehrerer vorheriger Ansprüchen **dadurch gekennzeichnet ist, dass**:
- die obengenannten Elemente (101 a, 101b, ..., usw.) der *magnetischen Plattform* (105) eine zylindrische Form haben und quer zur Längsrichtung des obengenannten Betriebskanals (104), in dem sie eingefügt wurden, angeordnet sind, so dass sie in seinem Inneren rollen können und so, dass daher eine reduzierte Reibung der *magnetischen Plattform* (105) während ihrer Verschiebung ermöglicht wird.

5. Gerät (100), das gemäß einem oder mehrerer vorheriger Ansprüchen **dadurch gekennzeichnet ist, dass**:
- die obengenannten Elemente (102a, 102b, ..., usw.) der *magnetischen Bahn* (107) eine zylindrische Form haben und koaxial zur Längsrichtung des obengenannten Endotrachealtubus (108), in dem sie eingefügt wurden, angeordnet sind, so dass die Neigung geändert werden kann, um den Biegungen desselben Endotrachealtubus (108) zu folgen, wenn dieser seinem Lauf in Kontakt mit der *magnetischen Plattform* (105) folgt.

6. Gerät (100), das gemäß einem oder mehrerer vorheriger Ansprüchen **dadurch gekennzeichnet ist, dass**:
- die obengenannte Trägerstruktur (103) der *magnetischen Plattform* (105) aus einer Reihe von Elementen besteht, die alle entsprechenden magnetischen Elemente (101a, 101b, ..., usw.), die steif in der Längsrichtung und gelenkig in der Querrichtung sind, miteinander verbinden; das gleiche Gefüge (103) endet im Teil, der dem Bediener am nächsten ist, mit einer gezahnten Oberfläche, die an die entsprechenden Zahnradgetriebe eines Zahnrades (120) angekoppelt ist; wenn der Bediener das Rad (120) rückwärts drehen lässt, schiebt sich die *magnetischen Plattform* (105) vorwärts tief in die Klinge, wenn der Bediener das Rad (120) vorwärts drehen lässt, schiebt sich die *magnetischen Plattform* (105) zurück zum Teil, der dem Bediener am nächsten ist.

7. Gerät (100), das gemäß einem oder mehrerer vorheriger Ansprüchen **dadurch gekennzeichnet ist, dass**:
- die obengenannte Trägerstruktur (106) der *magnetischen Bahn* (107) aus einer Reihe von Elementen besteht, die alle entsprechenden magnetischen Elemente (102a, 102b,..., usw.), die steif in der Längsrichtung und gelenkig in der Querrichtung sind, miteinander verbinden; wenn der Bediener das Gefüge (106) vorwärts drückt, schiebt sich die *magnetischen Bahn* (107) vorwärts tief in den Endotrachealtubus (108), wenn der Bediener das Gefüge (106) nach hinten zieht, tritt die *magnetischen Bahn* (107) aus dem Endotrachealtubus (108).

8. Gerät (100), das gemäß einem oder mehrerer vorheriger Ansprüchen **dadurch gekennzeichnet ist, dass**:
- das entfernte Endstück (122) der Klinge des Geräts (100) gelenkig ist und im Uhrzeigersinn und entgegen dem Uhrzeigersinn in Bezug auf einen Querstift (121) drehen kann; das letzte Element (123) der *magnetischen Plattform* (105) besteht aus einem magnetisch neutralen Material, z.B. Kunststoff, so dass ein Vorschub der *magnetischen Plattform* (105) es gegen die Wände des Endstückes (122) drückt und so, dass sie angehoben wird und somit die indirekte Verschiebung von Geweben und/oder anatomischen Teilen des Patienten ermöglicht; dasselbe entfernte Endstück (122) umfasst eine Rückstellfeder, die die Rückkehr des Endstückes (122) in seine Ausgangsposition ermöglicht, wenn die *magnetischen Plattform* (105) zurücktritt und das Element (123) daher stoppt, einen Druck auf die Wände auszuüben und auch dieses in seinen Ausgangsposition zurückkehrt; eine elastische Schutzmembran, die sich im Endteil des Betriebskanals (104) befindet, ermöglicht einerseits den kontrollierten Austritt von wenigstens zwei der Elemente der magnetischen Plattform (105) neben den distalen Endstücken des Betriebskanals (104) und gewährleistet andererseits die Sterilität der magnetischen Plattform (105), die so unverzüglich auf einer anderen Einwegklinge oder auf anderen Geräten, die diese Technologie verwenden, verwendet werden kann.

9. Gerät (100), das gemäß einem oder mehrerer vorheriger Ansprüchen **dadurch gekennzeichnet ist, dass**:
- die obengenannten magnetischen Elemente (101a, 101b, ..., usw.) der *magnetischen Plattform* (105) aus Permanentmagneten oder Elektromagneten bestehen, die eine geeignete Stromversorgungsschaltung mit entsprechenden Batterien und Steuerungen zum Einschalten, Einstellen und Ausschalten umfassen;
- die obengenannten magnetischen Elemente (102a, 102b, ..., etc.) der *magnetischen Bahn* (107) aus Permanentmagneten oder Elektromagneten bestehen, die eine geeignete Stromversorgungsschaltung mit entsprechenden Batterien und Steuerungen zum Einschalten, Einstellen und Ausschalten umfassen.

10. Gerät (100), das gemäß einem oder mehrerer vorheriger Ansprüchen **dadurch gekennzeichnet ist, dass** es ferner folgendes umfasst:
- eine erste Einweg-Verkleidung, vorzugsweise aus sterilisiertem Kunststoffmaterial, das die Klinge des Geräts (100) und die gesamte *magnetische Plattform* (105) verkleidet, so dass mögliche Infektionen und/oder Verunreinigungen vermieden werden; das Gerät (100) selbst kann Einweg sein;
- eine zweite Einweg-Verkleidung, vorzugsweise aus sterilisiertem Kunststoffmaterial, das die gesamte *magnetische Bahn* (107), die im inneren Teil des Endotrachealtubus (108) liegt, verkleidet, so dass mögliche Infektionen und/oder Verunreinigungen vermieden werden.

11. Gerät (100), das gemäß einem oder mehrerer vorheriger Ansprüchen **dadurch gekennzeichnet ist, dass**:
- der obengenannte Betriebskanal (104), in dem die *magnetische Plattform* (105) durchläuft, perfekt parallel zu einem optisch visuellen Kanal (128) ist, durch welchen visuelle Signale eines Bildes, das am distalen Endstück des Geräts (100) aufgenommen wurde, übertragen werden.

12. Gerät (100), das gemäß dem Anspruch 11 **dadurch gekennzeichnet ist, dass**:
- der obengenannte Betriebskanal (104) vom Sichtpunkt des Bedieners aus in Bezug auf den optisch visuellen Kanal (128) im rechten Teil liegt oder im linken Teil, um zum Beispiel etwaige linkshändige Bediener zu unterstützen.

13. Gerät (100), das gemäß dem Anspruch 11 **dadurch gekennzeichnet ist, dass**:
- sich der obengenannte Betriebskanal (104) und der obengenannte optisch visuelle Kanal (128) seitlich zueinander befinden mit dem Endotrachealtubus (108), der im Mittelraum untergebracht ist,
so dass die anatomischen Teile des Patienten besser geschützt sind, im Vergleich zur Situation, in der der Endotrachealtubus (108) die Zunge berühren kann und das Gewebe zwischen dem Tubus (108) selbst und den Elemente des Geräts (100) eingeklemmt werden kann, mit den daraus folgenden Risiken von Abschürfungen und inneren Verletzungen des Patienten.

14. Gerät (100), das gemäß dem Anspruch 6 **dadurch gekennzeichnet ist, dass**:
- die *magnetische Plattform* (105) vollständig aus dem Betriebskanal (104) entfernbar/herausziehbar ist, wenn die Trägerstruktur (103) nach vorheriger Verschiebung/Entfernung des Zahnradgetriebes (120) vom Bediener energisch gezogen/gespannt wird,
so dass das Entfernen des Geräts (100) aus dem Mund des Patienten einfacher und sicherer und schonender gemacht wird, sobald der Endotrachealtubus (108) korrekt in der Luftröhre positioniert wurde.

15. Gerät (100), das gemäß dem Anspruch 6 **dadurch gekennzeichnet ist, dass**:
- das besagte Zahnrad (120) mit einem Verriegelungssystem/sicheren System ausgestattet ist, dass das Zurückziehen und/oder den Vorschub der *magnetischen Plattform* (105) entlang dem Betriebskanal (104) unmöglich macht.

## Revendications

1. Dispositif (100) particulièrement adapté comme aide pour la procédure d'intubation trachéale d'un patient, et appliqué de préférence à la lame d'un laryngoscope. Le dispositif comprend :
- une première série d'éléments magnétiques (101a, 101b, ..., etc.) qui sont tous reliés l'un à l'autre par l'intermédiaire d'une première structure flexible/articulable de soutien (103) pour constituer une *plateforme magnétique* (105) proprement dite, libre de se déplacer sous le contrôle d'un opérateur le long de la direction longitudinale, en avant ou en arrière, à l'intérieur d'un canal opératif (104) appartenant au dispositif (100) ;
- une deuxième série d'éléments magnétiques (102a, 102b, ..., etc.) qui sont tous reliés l'un à l'autre par l'intermédiaire d'une deuxième structure flexible de soutien (106), pour constituer un *train magnétique* (107) proprement dit, inséré au préalable à l'intérieur d'un tube endotrachéal (108) ; les éléments (102a, 102b, ..., etc.) du *train magnétique* (107) ayant une polarité opposée par rapport aux éléments (101a, 101b, ..., etc.) de la *plateforme magnétique* (105), de façon à ce que de leur interaction se forme de façon continue une force magnétique d'attraction qui maintienne le tube endotrachéal (108) toujours en contact avec la *plateforme magnétique* (105),
de façon à ce que, sous le contrôle d'un opérateur, le tube endotrachéal (108) puisse bouger, en avant et en arrière, le long de tout le profil longitudinal du canal opératif (104), qui constitue donc un système de guidage et d'acheminement pour le tube endotrachéal (108), car ce dernier en suit les courbes et est orienté à la sortie avec l'extrémité distale (110) dans la direction tangente au profil du canal opératif (104), dans son dernier point de contact magnétique (109).

2. Dispositif (100), selon la revendication précédente, **caractérisé par le fait que** :
- la première série d'éléments magnétiques susmentionnée (101a, 101b, ..., etc.) est constituée entièrement d'éléments magnétiques, ou est constituée d'une alternance d'éléments magnétiques et d'éléments d'un matériel magnétiquement neutre, par ex. plastique, ceux-ci ayant la seule fonction d'éloigner mécaniquement les éléments de la *plateforme magnétique* (105) susdite.

3. Dispositif (100), selon la revendication précédente, **caractérisé par le fait que** :
- la deuxième série d'éléments magnétiques susmentionnée (102a, 102b, ..., etc.) est constituée entièrement par des éléments magnétiques, ou est constituée d'une alternance d'éléments magnétiques et d'éléments d'un matériel magnétiquement neutre, par ex. plastique, ceux-ci ayant la seule fonction d'éloigner mécaniquement les éléments du *train magnétique* (107) susdit.

4. Dispositif (100), selon une ou plusieurs des revendications précédentes, **caractérisé par le fait que** :
- les éléments susdits (101a, 101b, ..., etc.) de la *plateforme magnétique* (105) ont une forme cylindrique et sont disposés de façon transversale par rapport à la direction longitudinale du canal opératif susdit (104) dans lequel ceux-ci sont insérés, de façon à pouvoir rouler à l'intérieur de celui-ci, et de façon à permettre donc un frottement réduit de la *plateforme magnétique* (105) lors de son déplacement.

5. Dispositif (100), selon une ou plusieurs des revendications précédentes, **caractérisé par le fait que** :
- les éléments susdits (102a, 102b, ..., etc.) du *train magnétique* (107) ont une forme cylindrique et sont disposés de façon coaxiale par rapport à la direction longitudinale du tube endotrachéal susdit (108) dans lequel ceux-ci sont insérés, de façon à pouvoir changer l'inclinaison pour suivre les courbes de ce même tube endotrachéal (108) lorsque celui-ci suit son parcours en contact avec la *plateforme magnétique* (105).

6. Dispositif (100), selon une ou plusieurs des revendications précédentes, **caractérisé par le fait que** :
- la structure de soutien susdite (103) de la *plateforme magnétique* (105) est constituée d'une série d'éléments qui relient tous les éléments magnétiques respectifs (101a, 101b, ..., etc.) qui sont rigides dans la direction longitudinale et articulés dans la direction transversale ; cette même structure (103) termine dans la partie la plus proche de l'opérateur avec une superficie supérieure dentée qui est accouplée aux engrenages respectifs d'une roue dentée (120) ; lorsque l'opérateur fait tourner la roue (120) vers l'arrière, la *plateforme magnétique* (105) avance en profondeur dans la lame, tandis que lorsque l'opérateur fait tourner la roue (120) vers l'avant, la *plateforme magnétique* (105) s'arrête vers la partie la plus proche de l'opérateur.

7. Dispositif (100), selon une ou plusieurs des revendications précédentes, **caractérisé par le fait que** :
- la structure de soutien susdite (106) du *train magnétique* (107) est constituée d'une série d'éléments qui relient tous les éléments magnétiques respectifs (102a, 102b, ..., etc.) qui sont rigides dans la direction longitudinale et articulés dans la direction transversale ; lorsque l'opérateur pousse la structure (106) vers l'avant, le *train magnétique* (107) avance en profondeur dans le tube endotrachéal (108), tandis que lorsque l'opérateur tire la structure (106) vers l'arrière, le *train magnétique* (107) recule du tube endotrachéal (108).

8. Dispositif (100), selon une ou plusieurs des revendications précédentes, **caractérisé par le fait que** :
- l'extrémité distante (122) de la lame du dispositif (100) est articulée et peut tourner dans le sens des aiguilles d'une montre ou dans le sens contraire par rapport à un axe transversal (121) ; le dernier élément (123) de la *plateforme magnétique* (105) est constitué d'un matériel magnétiquement neutre, par ex. plastique, de façon à ce qu'un avancement de la *plateforme magnétique* (105) le fasse appuyer contre les parois de l'extrémité (122) et de façon à la soulever et à permettre ainsi le déplacement indirect de tissus et/ou de parties anatomiques du patient ; cette même extrémité distante (122) comprend un ressort qui permet le retour de l'extrémité (122) dans sa position initiale, au moment où la *plateforme magnétique* (105) recule et l'élément (123) arrête donc d'exercer une pression sur les parois et retourne lui aussi dans sa position initiale ; une membrane élastique de protection, placée dans la partie terminale du canal opératif (104), permet d'un côté la sortie contrôlée d'au moins deux des éléments de la plateforme magnétique (105), outre l'extrémité distale du canal opératif (104), et garantit d'un autre côté la stérilité de la plateforme magnétique (105), qui peut ainsi être immédiatement utilisée sur une autre lame jetable ou sur d'autres dispositifs qui utilisent cette technologie.

9. Dispositif (100), selon une ou plusieurs des revendications précédentes, **caractérisé par le fait que** :
- les éléments magnétiques susdits (101a, 101b, ..., etc.) de la *plateforme magnétique* (105) sont constitués d'aimants permanents ou d'électroaimants comprenant un circuit d'alimentation électrique à cet effet, avec les piles relatives et les commandes d'allumage, de réglage et d'arrêt ;
- les éléments magnétiques susdits (102a, 102b, ..., etc.) du *train magnétique* (107) sont constitués d'aimants permanents ou d'électroaimants comprenant un circuit d'alimentation électrique à cet effet, avec les piles relatives et les commandes d'allumage, de réglage et d'arrêt ;

10. Dispositif (100), selon une ou plusieurs des revendications précédentes, **caractérisé par** le fait de comprendre en outre :
- un premier revêtement jetable, de préférence en matériel plastique stérilisé, qui recouvrira la lame du dispositif (100) et toute la *plateforme magnétique* (105), de façon à éviter d'éventuelles infections et/ou contaminations ; le dispositif (100) peut être jetable lui aussi ;
- un deuxième revêtement jetable, de préférence en matériel plastique stérilisé, qui recouvrira tout le *train magnétique* (107), situé dans la partie intérieure du tube endotrachéal (108), de façon à éviter d'éventuelles infections et/ou contaminations.

11. Dispositif (100), selon une ou plusieurs des revendications précédentes, **caractérisé par le fait que** :
- le canal opératif susdit (104), dans lequel glisse la *plateforme magnétique* (105), est parfaitement parallèle à un canal optique visuel (128) à travers lequel sont transmis les signaux visuels d'une image photographiée à l'extrémité distale du dispositif (100).

12. Dispositif (100), selon la revendication 11, **caractérisé par le fait que** :
- le canal opératif susdit (104) est situé dans la partie droite, du point de vue de l'opérateur, par rapport à un canal optique visuel (128), ou dans la partie gauche, par exemple pour aider les éventuels opérateurs gauchers.

13. Dispositif (100), selon la revendication 11, **caractérisé par le fait que** :
- le canal opératif susdit (104) et le canal optique visuel susdit (128) se trouvent latéralement l'un par rapport à l'autre, avec le tube endotrachéal (108) dans l'espace central,
de façon à ce que les parties anatomiques du patient soient plus protégées par rapport à des situations lors desquelles le tube endotrachéal (108) pourrait toucher la langue, et les tissus pourraient rester encastrés entre ce même tube (108) et les éléments du dispositif (100), avec des risques conséquents d'abrasions et de lacérations internes pour le patient.

14. Dispositif (100), selon la revendication 6, **caractérisé par le fait que** :
- la *plateforme magnétique* (105) est entièrement extractible du canal opératif (104), lorsque la structure de soutien (103) est tirée énergiquement par l'opérateur, après le déplacement/l'enlèvement de l'engrenage (120),
de façon à faciliter et à sécuriser l'enlèvement du dispositif (100) de la bouche du patient une fois que le tube endotrachéal (108) a été placé correctement dans la trachée.

15. Dispositif (100), selon la revendication 6, **caractérisé par le fait que** :
- cette roue dentée (120) est dotée d'un système de blocage/sécurité qui rend le recul ou l'avancement de la *plateforme magnétique* (105) impossible le long du canal opératif (104) lorsque ce système est activé.
